# EUROPEAN PATENT APPLICATION

(11) **EP 3 508 855 A1**
(43) Date of publication of application: **10.07.2019**
(21) Application number: 19150257.4
(22) Date of filing: 03.01.2019
(51) Int. Cl.: G01N 33/574

(54) **METHOD FOR THE SELECTION OF PATIENTS AND KIT**

(30) Priority: 05.01.2018 US 201862614029 P
(71) Applicant: Allianz Pharmascience Ltd, 10682 Taipei (TW)
(72) Inventor: Chan, Hardy W, Redwood City, CA California 94065 (US); Chen, Tzu Chi, 116 Taipei City (TW)
(74) Representative: Fuchs Patentanwälte Partnerschaft mbB

(57) **Abstract**

The invention provides a method for selecting a patient who may respond to a compound having at least one (substituted phenyl)-propenal moiety, which comprises contacting the compound with a sample derived from the patient and determining whether the compound decreases the amount of a protein selected from AR, c-MYC, Aurora-A, mutated p53, CDK4, or Her2 or whether the compound increases the amount of reactive oxygen species in the sample, wherein decrease of the protein or increase of the reactive oxygen species indicates that the patient may respond to the compound. The invention also provides a kit, a method for identifying a candidate compound which may treat an androgen receptor associated medical condition, and a candidate compound identified by the method as mentioned above.

## Description

### FIELD OF THE INVENTION

This invention relates to a detection technique. More specifically, the invention relates to a method for selecting a patient who may respond to a compound having at least one (substituted phenyl)-propenal moiety.

### BACKGROUND OF THE INVENTION

Targeted therapy is a mainstream approach to research disease treatment nowadays. According to the National Institutes of Health, theargeted therapy is the foundation of precision medicine. It is a type of cancer treatment that targets the changes in cancer cells that help them grow, divide, and spread. As researchers learn more about the cell changes that drive cancer, they are better able to design promising therapies that target these changes or block their effects."

It is well known that chemotherapy usually causes severe side effects. Moreover, the cost of chemotherapy can be very expensive. However, the effects of chemotherapy are not satisfactory. As published in the Journal of Clinical Oncology in 2004 (The Contribution of Cytotoxic Chemotherapy to 5-year Survival in Adult Malignancies. Clin Oncol (R Coll Radiol). 2004 Dec;16(8):549-560), the overall contribution of curative and adjuvant cytotoxic chemotherapy to 5-year survival in adults was estimated to be 2.3% in Australia and 2.1% in the USA. Therefore, a screening method for selecting a patient who may respond to a specific drug is regarded to be as important as the treatment itself.

### SUMMARY OF THE INVENTION

The invention is to provide a method for the selection of patients who may respond to a drug for increasing the effective percentage of chemotherapy and optimize medical expenses.

The invention provides a method for selecting a patient who may respond to a compound according to formula I, IIa, IIb, IIc, III, IV, V, VI, VII or VIII, which comprises contacting the compound with a sample derived from the patient and determining whether the compound decreases the amount of a protein selected from AR, c-MYC, Aurora-A, mutated p53, CDK4, or Her2 or whether the compound increases the amount of reactive oxygen species in the sample, wherein decrease of the protein or increase of the reactive oxygen species indicates that the patient may respond to the compound; 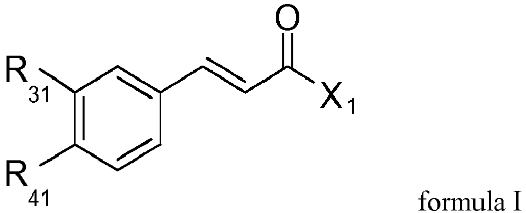 wherein in formula I:
R₃₁ and R₄₁ are each independently selected from the group consisting of alkoxy, hydroxy, and hydrogen; and
X₁ is selected from the group consisting of hydroxy, alkoxy, ethyl propionate, ethyl methyl carbonate, and carbonyl alkyl; 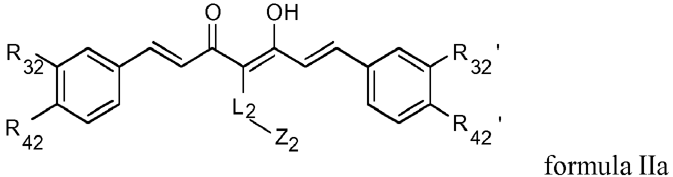 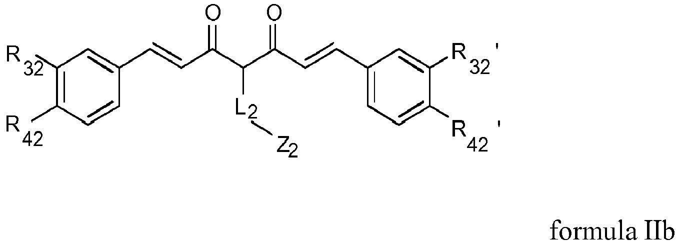
wherein in formula IIa and formula IIb:
R₃₂, R₄₂, R₃₂', and R₄₂' are independently selected from the group consisting of -H, -OH, and -OCH₃;
L₂ is a C0 - C8 alkylene; or L₂ is an unsaturated alkenylene or alkynl when Z₂ is nothing; Z₂ is selected from the group consisting of -H, -OH, an aromatic ring, a cycloalkyl, -COR₁₂, -CO₂R₁₂, -CONR₁₂R₂₂, -NR₁₂R₂₂, -C(X₂)₃, wherein R₁₂ and R₂₂ are independently selected from the group consisting of -H, -CH₃, and -C₂H₅; and
X₂ is a halogen atom selected from the group consisting of -F, -Cl, and -Br; 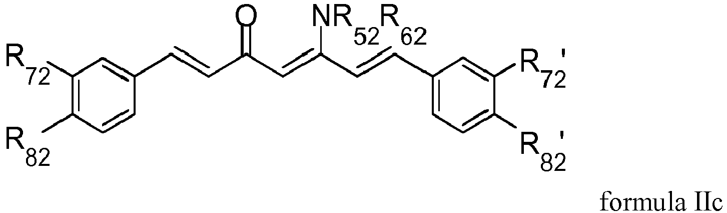
wherein in formula IIc:
R₇₂, R₈₂, R₇₂', and R₈₂' are independently selected from the group consisting of -H, -OH, and -OCH₃; and
R₅₂, and R₆₂ are independently selected from the group consisting of -H, -CH₃, -C₂H₅, a substituted aryl and a substituted benzyl group; 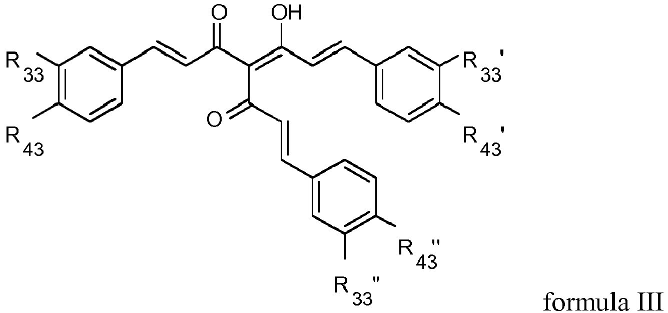
wherein in formula III:
R₃₃, R₄₃, R₃₃', R₄₃', R₃₃", and R₄₃" are each independently selected from the group consisting of alkoxy, hydroxy, and hydrogen; 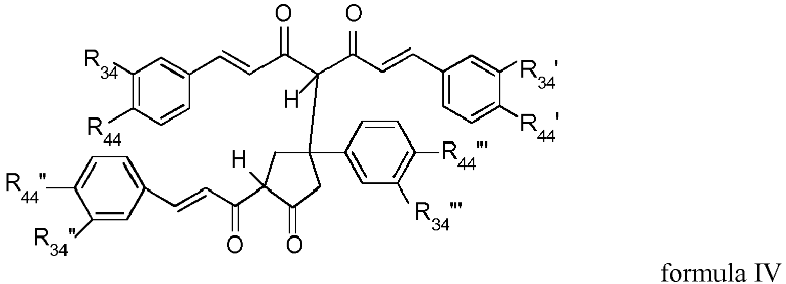 wherein in formula IV:
R₃₄, R₄₄, R₃₄', R₄₄', R₃₄", R₄₄", R₃₄'", and R₄₄'" are each independently selected from the group consisting of alkoxy, hydroxy, and hydrogen; 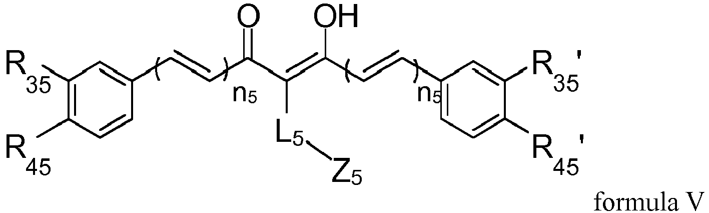 wherein in formula V:
each "ns" is independently 1, 2, or 3;
R₃₅, R₄₅, R₃₅', and R₄₅' are independently selected from the group consisting of -H, -OH, and -OCH₃;
L₅-Z₅ is absent; or
L₅-Z₅ exists, and L₅ is selected from the group consisting of a C0-C8 alkylene, an unsaturated alkenylene, and alkynl when Z₅ is absent;
Z₅ is selected from the group consisting of -H, -OH, an aromatic ring, a cycloalkyl, -COR₁₅, -CO₂R₁₅, -CONR₁₅R₂₅, -NR₁₅R₂₅, -C(X₅)₃;
R₁₅ and R₂₅ are independently selected from the group consisting of -H, -CH₃, and -C₂H₅; and
X₅ is a halogen atom selected from the group consisting of -F, -Cl, and -Br; 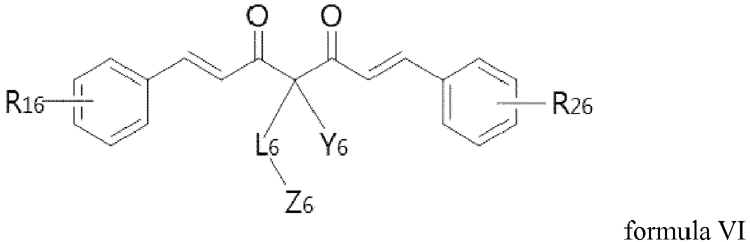 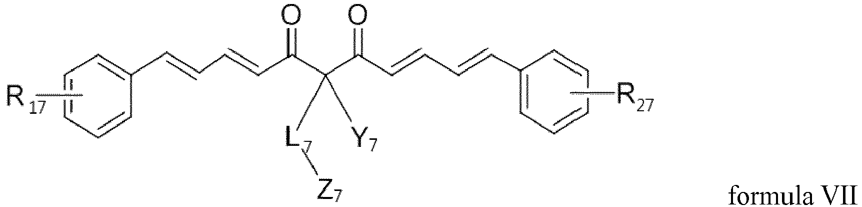
wherein in formula VI and formula VII:
R₁₆, R₁₇, R₂₆ and R₂₇ are mono- or di-substituted groups and independently selected from the group consisting of a methoxy group, a hydroxyl group, an alkyl sulfonyl group;
L₆ and L₇ are each selected from the group consisting of carbonyl, alkylene, alkenylene, and alkynl when Z₆ and Z₇ are absent;
Z₆ and Z₇ are each selected from the group consisting of -H, -OH, a substituted styrenyl, an aromatic ring, a cycloalkyl, -COR₃₆, -CONR₃₆R₄₆, and -C(X₆)₃, wherein R₃₆ and R₄₆ are each selected from the group consisting of -H, -CH₃, or -Cₙ₆H₂ₙ₆₊₁,, a heterocyclic, and a heteroaryl moiety; or R₃₆ and R₄₆ together form a heterocyclic ring; or
Z₆ and Z₇ are each COOR₆; wherein R₆ is selected from the group consisting of -H, -CH₃, and -Cₙ₇H₂ₙ₇₊₁; or
Z₆ and Z₇ are each a cycloalkyl when Y₇ is not H;
n₆ and n₇ are independently an integer from 2 to 4;
X₆ is selected from the group consisting of -F, -Cl, and -Br;
Y₆ and Y₇ are each H when L is an alkylene and Z₆ and Z₇ are -CONR₃₇R₄₇, R₃₇ and R₄₇ are each selected from the group consisting of -H, a hetercyclic, a heteroaryl,and a cycloalkyl; or
Y₆ and Y₇ are each selected from the group consisting of C1 - C3 alkyl, -F, Cl, and Br; and 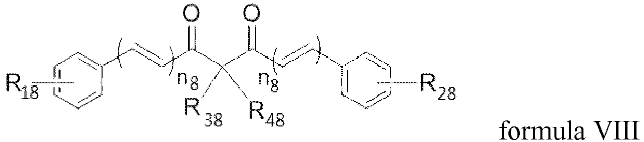
R₁₈ and R₂₈ are mono- or di-substituted groups and independently selected from the group consisting of a methoxy group, a hydroxyl group, and an alkyl sulfonyl group;
R₃₈ is selected from the group consisting of 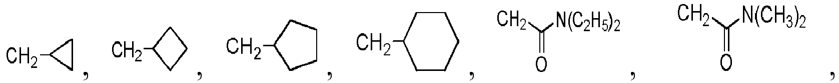 (CH₂)₃CH₃, 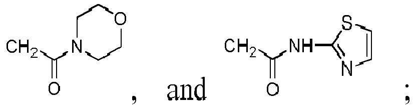 and
R₄₈ is selected from the group consisting of CH₃, H, F and Cl; and
n₈ is 1 or 2.

The invention also provides a kit comprising a compound according to formula I, IIa, IIb, IIc, III, IV, V, VI, VII or VIII and a detecting molecule for detecting the amount of a protein selected from AR, c-MYC, Aurora-A, mutated p53, CDK4, or Her2, or a detecting molecule for detecting the amount of reactive oxygen species.

The invention also provides a method for identifying a candidate compound which may treat an androgen receptor associated medical condition, which comprises contacting the candidate compound with a sample derived from a patient suffering the androgen receptor associated medical condition and determining whether the candidate compound decreases the amount of a protein selected from AR, c-MYC, Aurora-A, mutated p53, CDK4, or Her2 or determining whether the compound increases the amount of reactive oxygen species in the sample, wherein decrease of the protein or increase of the reactive oxygen species indicates that the candidate compound may treat the androgen receptor associated medical condition.

The invention also provides a kit comprising a sample derived from a patient suffering the androgen receptor associated medical condition and a detecting molecule for detecting the amount of a protein selected from AR, c-MYC, Aurora-A, mutated p53, CDK4, or Her2 or a detecting molecule for detecting the amount of reactive oxygen species.

The invention also provides a candidate compound identified by the method as mentioned above.

The present invention is described in detail in the following sections. Other characteristics, purposes and advantages of the present invention can be found in the detailed description and claims.

### BRIEF DESCRIPTION OF THE DRAWINGS

FIG. 1. JM17 showed reduction of AR and c-Myc proteins in CWR22RV1, a prostate cancer cell-line. The protein expressions of AR, AR-V7, c-MYC and LC3 were analyzed by western blotting with serial doses of JM17 for 6 hr (left) and 24 hr (right).
FIG. 2. JM17 enhanced AR protein degradation. CWR22RV1 cells were treated with cycloheximide (CHX) with or without 5 µM JM17 and harvested at indicated time points for western blotting. The quantification of AR band intensities, normalized to GAPDH, is shown on the right.
FIG. 3. JM17 enhanced c-MYC protein degradation. CWR22RV1 cells were treated with cycloheximide (CHX) with or without 5 µM JM17 and harvested at indicated time points for western blotting. The quantification of c-MYC band intensities, normalized to GAPDH, is shown on the right.
FIG. 4. JM17 enhanced Aurora A protein degradation. CWR22RV1 cells were treated with cycloheximide (CHX) with or without 5 µM JM17 and harvested at indicated time points for western blotting of Aurora A.
FIG. 5. JM17 triggered the ROS mediated protein degradation in prostate cancer cells. CWR22RV1 cells were treated with DMSO control or JM17 at the dose of 1.25, 2.5, and 5 µM. The ROS level was detected at 10, 30, or 60 min after JM17 was added (FIG. 5a). The expression level of AR, AR-V7, c-Myc and p62 was detected via western blot (FIG. 5b).
FIG. 6. JM17 suppressed BT-474 cell growth coupling with the decrease of protein expression on Her2, AR, c-MYC, mutant p53 and CDK4. The JM17 significantly reduced cell growth of BT-474 for 48 hr-treatment (FIG. 6a). The JM17 decreased the protein expression of Her2, AR, c-MYC, mutant p53 and CDK4 at the dose of 1 µM (FIG. 6b). **, p < 0.01, compared with the control group.
FIG. 7. JM17 enhanced CDK4 protein degradation. MDA-MB-231 cells were treated with cycloheximide (CHX) with or without 2.5 µM JM17 and harvested at indicated time points for western blotting for CDK4 protein.
FIG. 8. JM17 showed the reduction of Her2, AR, c-Myc, p53, and CDK4 proteins in BT-474, a breast cancer cell-line. The expressions of indicated proteins were analyzed by western blotting with serial doses of JM17.
FIG. 9. JM17 inhibited tumor growth in breast xenograft model. BT-474 tumor cells were inoculated subcutaneously on mice. The JM17 was given by I.P. injection daily at 5, 25 or 40 mg/kg. Tumor volume was measured weekly by caliper (left). Variations of body weight were monitored weekly after JM17 injection (right).
FIG. 10. JM17 diminished Her2, AR, c-MYC, p53 expression in xenograft. After mouse sacrifice as described in FIG. 6, the protein expression of tumors was measured by immunoblotting with indicated antibodies.
FIG. 11. JM17 significantly reduced the growth rate of MDA-MB-231 tumor in mice xenograft model. The upper panel shows the average size of subcutaneous tumors after treatment with the vehicle (as the control), 5 mg/kg or 20 mg/kg of JM17, 3 times a week for 28 days. The JM17 significantly reduced the tumor size at the dose of 20 mg/kg. The tumors were isolated for protein extraction at 28 days after the vehicle, 5 mg/kg or 20 mg/kg of JM17 treatment (the lower panel).

### DETAILED DESCRIPTION OF THE INVENTION

Cancer drug sensitivity screening with biomarker(s) is a useful tool to select cancer patients for effective cancer treatment.

The invention provides a method for selecting a patient who may respond to a compound having at least one (substituted phenyl)-propenal moiety according to formula I, IIa, IIb, IIc, III, IV, V, VI, VII or VIII, which comprises contacting the compound with a sample derived from the patient and determining whether the compound decreases the amount of a protein selected from AR, c-MYC, Aurora-A, mutated p53, CDK4, or Her2 or whether the compound increases the amount of reactive oxygen species in the sample, wherein decrease of the protein or increase of the reactive oxygen species indicates that the patient may respond to the compound.

The term "(substituted phenyl)-propenal moiety" as used herein refers to a composition including a phenyl group having attached thereto a propenal moiety (when m equals 1) and an alkoxy or hydroxy moiety, or an alkyl or substituted alkyl moiety. The substitutions may be positioned meta or para or ortho with respect to the propenal moiety as used herein and refers to a general formula 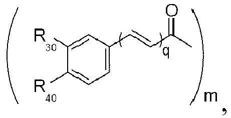 where q may be any number of 1, 2, 3 or 4; and m may be any number of 1, 2, 3, 4, or more.

The term "alkyl" as used herein refers to a straight or branched hydrocarbon chain radical consisting solely of carbon and hydrogen atoms, containing no unsaturation, having from one to ten carbon atoms, and which is attached to the rest of the molecule by a single bond, e.g. methyl, ethyl, n-propyl, 1-methylethyl (iso-propyl), n-butyl, n-pentyl, 1,1-dimethylethyl (t-butyl), and the like.

The term "alkenyl" as used herein refers to a straight or branched hydrocarbon chain radical consisting solely of carbon and hydrogen atoms, containing at least one double bond, having from two to ten carbon atoms, and which is attached to the rest of the molecule by a single bond or a double bond, e.g. ethenyl, prop-1-enyl, pent-1-enyl, penta-1,4-dienyl, and the like.

The term "alkenylene" as used herein refers to a straight or branched hydrocarbon chain which contains a carbon-to-carbon double bond and is represented by the formula CₚH₂ₚ₋₂, wherein hydrogen may be replaced by an additional carbon-to-carbon double bond or a monovalent substituent, e.g. ethenylene, prop-1-enylene and the like.

The term "alkoxy" as used herein refers to the radical having the formula -OR wherein R is an alkyl, haloalkyl or cycloalkyl. An "optionally substituted alkoxy" refers to the radical having the formula -OR' wherein R' is an optionally substituted alkyl as described herein.

The term "alkynl" as used herein refers to a straight or branched hydrocarbon chain radical consisting solely of carbon and hydrogen atoms, containing at least one triple bond, having from two to ten carbon atoms, and which is attached to the rest of the molecule by a single bond or a triple bond, e.g. ethynyl, prop-1-ynyl, but-1-ynyl, pent-1-ynyl, pent-3-ynyl and the like.

The term "aryl" as used herein refers to a radical of carbocyclic ring system wherein at least one of the rings is aromatic. The aryl may be fully aromatic or may contain an aromatic ring in combination with a non-aromatic ring. A "biaryl system" is a compound that includes at least two aryl groups.

The term "cycloalkyl" as used herein refers to a stable monovalent monocyclic or bicyclic hydrocarbon radical consisting solely of carbon and hydrogen atoms, having from three to ten carbon atoms, and which is saturated and attached to the rest of the molecule by a single bond, e.g. cyclopropyl, cyclobutyl, cyclopentyl, cyclohexyl, and the like.

The term "di-ketone bridge," or "ketone-enol bridge" as used herein, refers to a straight or branched hydrocarbon chain including two ketones or an enol positioned in close proximity to a ketone respectively. The "di-ketone bridge" or "ketone-enol bridge" is positioned between at least two aryl moieties.

The term "hydroxyalkyl" as used herein refers to a straight or branched hydroxy substituted hydrocarbon chain radical having from one to ten carbon atoms, e.g. -CH₂OH, -(CH₂)₂OH and the like.

The compound of formula I is shown below: 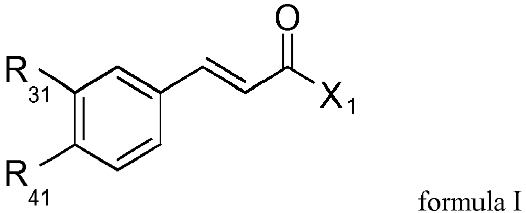 wherein in formula I:
R₃₁ and R₄₁ are each independently selected from the group consisting of alkoxy, hydroxy, and hydrogen; and
X₁ is selected from the group consisting of hydroxy, alkoxy, ethyl propionate, ethyl methyl carbonate, and carbonyl alkyl.

In some embodiments the compound has a formula selected from the group consisting of monomers 1, 3, 5, 6 and 7. These monomers are provided below:

### Monomers :

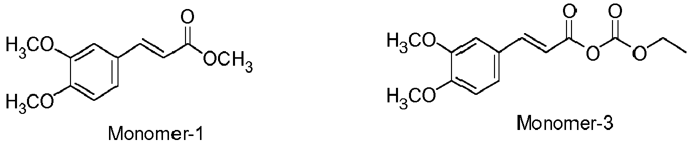

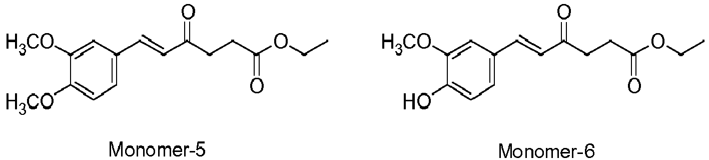

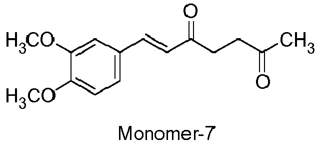

In various embodiments, derivatives of the above referenced monomers having biological activity are also provided. The derivatives may have substitutions at one or more positions to increase one or more characteristics such as activity, solubility and the like. Such derivatives may modulate the dipole moment of a compound and may result in a composition that is more or less hydrophobic or hydrophilic.

The compounds of formula IIa and formula IIb are shown below: 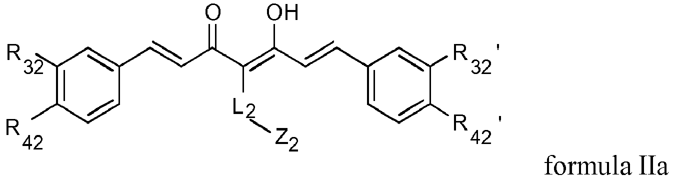 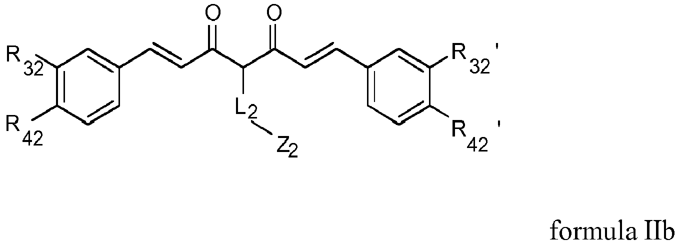 wherein in formula IIa and formula IIb:
R₃₂, R₄₂, R₃₂', and R₄₂' are independently selected from the group consisting of -H, -OH, and -OCH₃;
L₂ is a C0 - C8 alkylene; or L₂ is an unsaturated alkenylene or alkynl when Z₂ is nothing;
Z₂ is selected from the group consisting of -H, -OH, an aromatic ring, a cycloalkyl, -COR₁₂, -CO₂R₁₂, -CONR₁₂R₂₂, -NR₁₂R₂₂, -C(X₂)₃, wherein R₁₂ and R₂₂ are independently selected from the group consisting of -H, -CH₃, and -C₂H₅; and
X₂ is a halogen atom selected from the group consisting of -F, -Cl, and -Br; and
further wherein formula IIa and IIb are the equilibrate tautomers as a common phenomenon of diketone.

In some embodiments the compound is selected from the group consisting of II-1, II-2, II-3, II-4 and II-5. The formulas are:

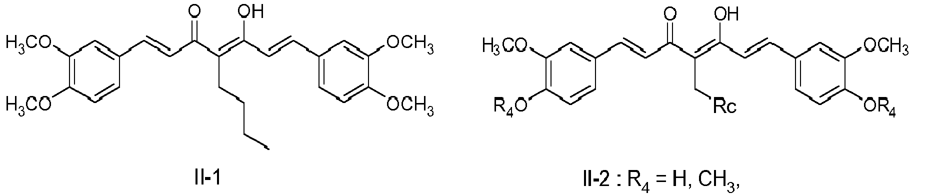

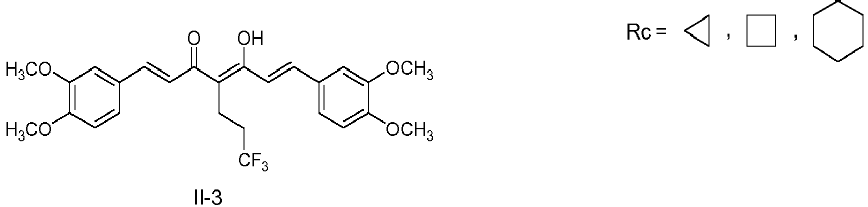

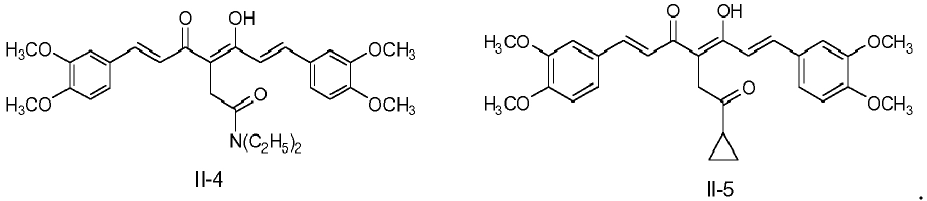

The compound of formula IIc is shown below: 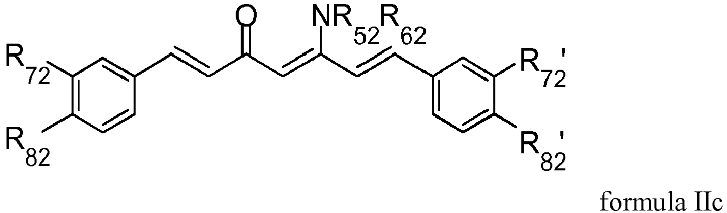 wherein in formula IIc:
R₇₂, R₈₂, R₇₂', and R₈₂' are independently selected from the group consisting of -H, -OH, and -OCH₃; and
R₅₂, and R₆₂ are independently selected from the group consisting of -H, -CH₃, -C₂H₅, a substituted aryl and a substituted benzyl group.

The compound of formula III is shown below: 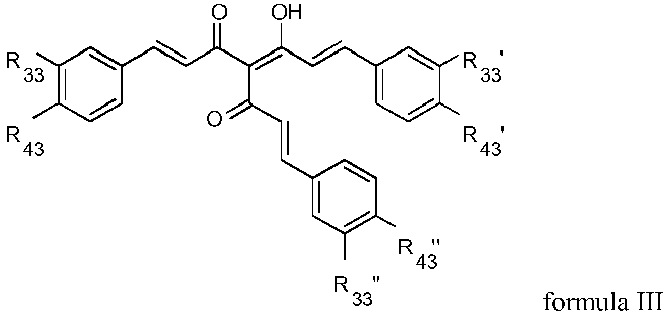 wherein in formula III:
R₃₃, R₄₃, R₃₃', R₄₃', R₃₃", and R₄₃" are each independently selected from the group consisting of alkoxy, hydroxy, and hydrogen.

In some embodiments, the compound includes the formula III-1 or III-2, which is:

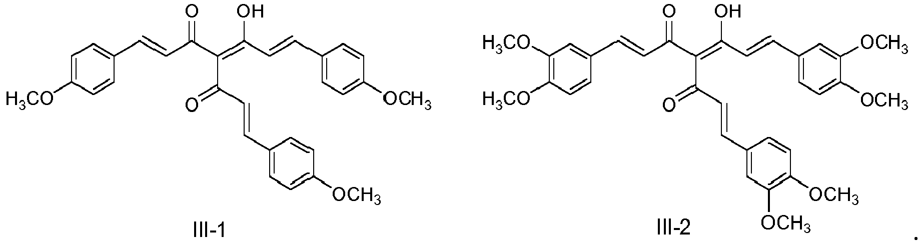

The compound of formula IV is shown below: 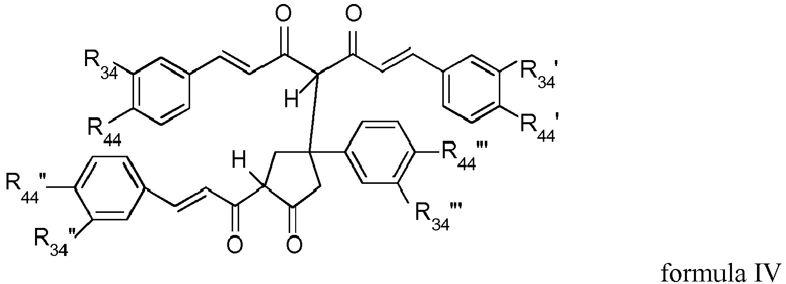 wherein in formula IV:
R₃₄, R₄₄, R₃₄', R₄₄', R₃₄", R₄₄", R₃₄'", and R₄₄'" are each independently selected from the group consisting of alkoxy, hydroxy, and hydrogen.

In some embodiments the compound includes the formula IV-1:

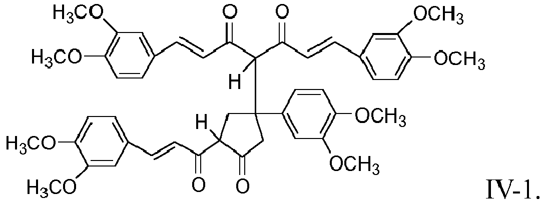

The compound of formula V is shown below: 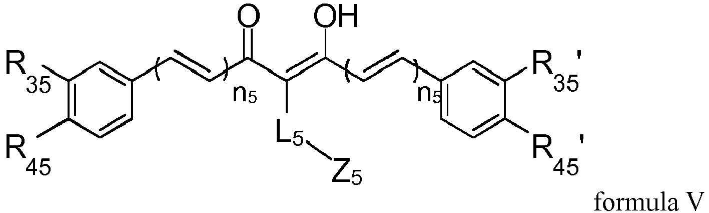 wherein in formula V:
each "n₅" is independently 1, 2, or 3;
R₃₅, R₄₅, R₃₅', and R₄₅' are independently selected from the group consisting of -H, -OH, and -OCH₃;
L₅-Z₅ is absent; or
L₅-Z₅ exists, and L₅ is selected from the group consisting of a C0-C8 alkylene, an unsaturated alkenylene, and alkynl when Z₅ is absent;
Z₅ is selected from the group consisting of -H, -OH, an aromatic ring, a cycloalkyl, -COR₁₅, -CO₂R₁₅, -CONR₁₅R₂₅, -NR₁₅R₂₅, -C(X₅)₃;
R₁₅ and R₂₅ are independently selected from the group consisting of -H, -CH₃, and -C₂H₅; and
X₅ is a halogen atom selected from the group consisting of -F, -Cl, and -Br.

In some embodiments the compound is provided according to formula V-1 or V-2:

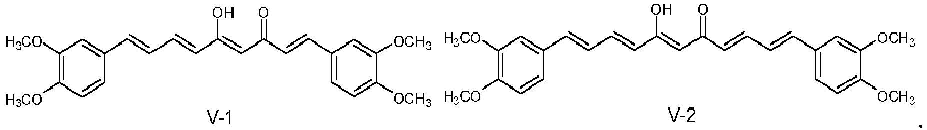

The compounds of formula VI and formula VII are shown below: 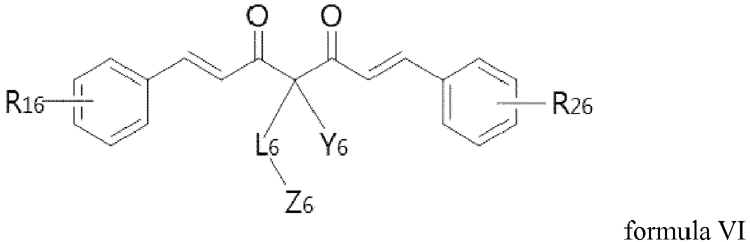 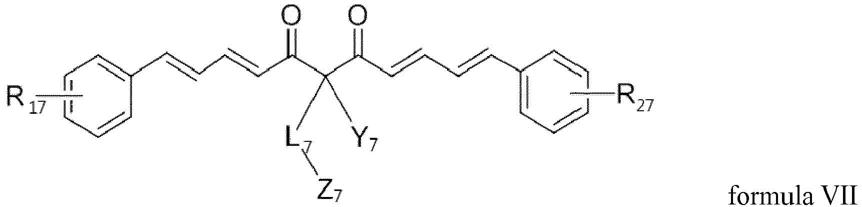 wherein in formula VI and formula VII:
R₁₆, R₁₇, R₂₆ and R₂₇ are mono- or di-substituted groups and independently selected from the group consisting of a methoxy group, a hydroxyl group, an alkyl sulfonyl group;
L₆ and L₇ are each selected from the group consisting of carbonyl, alkylene, alkenylene, and alkynl when Z₆ and Z₇ are absent;
Z₆ and Z₇ are each selected from the group consisting of -H, -OH, a substituted styrenyl, an aromatic ring, a cycloalkyl, -COR₃₆, -CONR₃₆R₄₆, and -C(X₆)₃, wherein R₃₆ and R₄₆ are each selected from the group consisting of -H, -CH₃, or -Cₙ₆H₂ₙ₆₊₁,, a heterocyclic, and a heteroaryl moiety; or R₃₆ and R₄₆ together form a heterocyclic ring; or
Z₆ and Z₇ are each COOR₆; wherein R₆ is selected from the group consisting of -H, -CH₃, and -Cₙ₇H₂ₙ₇₊₁; or
Z₆ and Z₇ are each a cycloalkyl when Y₇ is not H;
n₆ and n₇ are independently an integer from 2 to 4;
X₆ is selected from the group consisting of -F, -Cl, and -Br;
Y₆ and Y₇ are each H when L is an alkylene and Z₆ and Z₇ are -CONR₃₇R₄₇, R₃₇ and R₄₇ are each selected from the group consisting of -H, a hetercyclic, a heteroaryl,and a cycloalkyl; or
Y₆ and Y₇ are each selected from the group consisting of C1 - C3 alkyl, -F, Cl, and Br.

The compound of formula VIII is shown below: 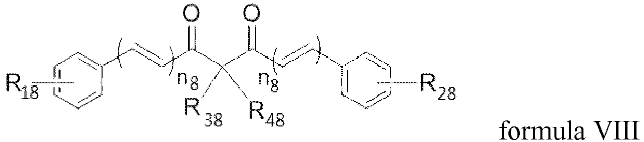
R₁₈ and R₂₈ are mono- or di-substituted groups and independently selected from the group consisting of a methoxy group, a hydroxyl group, and an alkyl sulfonyl group;
R₃₈ is selected from the group consisting of 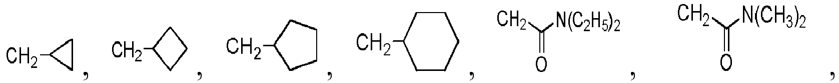 (CH₂)₃CH₃, 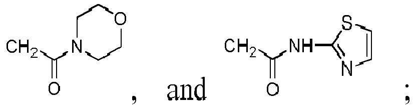 and
R₄₈ is selected from the group consisting of CH₃, H, F and Cl; and
n₈ is 1 or 2.

In one preferred embodiment of the invention, the compounds including 4,4-disubstituted 1,7-bis-(3,4-dimethoxyphenyl)-hepta-1,6-diene-3,5-dione and 6,6-disubstituted 1,11-bis(substituted phenyl)-undeca-1,3,8,10-tetraene-5,7-dione scaffolds are as shown in Table 1.

**Table 1:**

| | | | | | | |
|---|---|---|---|---|---|---|
| 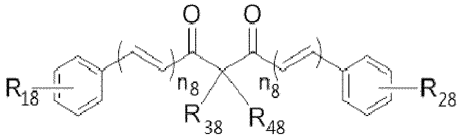 | | | | | | |

| **Compound ID** | **R₁₈** | **R₂₈** | **R₃₈** | **R₄₈** | **n₈** | **formula** |
|---|---|---|---|---|---|---|
| 1 | 3'4'-OCH₃ | 3'4'-OCH₃ | 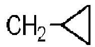 | CH₃ | 1 | C₂₈H₃₂O₆ 464.55 |
| 2 | 3'4'-OCH₃ | 3'4'-OCH₃ | 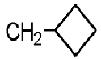 | CH₃ | 1 | C₂₉H₃₄O₆ 478.5767 |
| 3 | 3'4'-OCH₃ | 3'4'-OCH₃ | 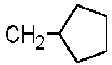 | H | 1 | C₂₉H₃₄O₆ 478.58 |
| 4 | 3'4'-OCH₃ | 3'4'-OCH₃ | 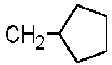 | CH₃ | 1 | C₃₀H₃₆O₆ 492.60 |
| 5 | 3'4'-OCH₃ | 3'4'-OCH₃ | 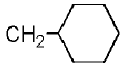 | CH₃ | 1 | C₃₁H₃₈O₆ 506.63 |
| 6 | 3'4'-OCH₃ | 3'4'-OCH₃ | 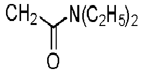 | CH₃ | 1 | C₃₀H₃₇NO₇ 523.62 |
| 7 | 3'4'-OCH₃ | 3'4'-OCH₃ | 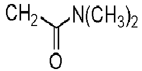 | CH₃ | 1 | C₂₈H₃₃NO₇ 495.56 |
| 8 | 3'4'-OCH₃ | 3'4'-OCH₃ | (CH₂)₃CH₃ | CH₃ | 1 | C₂₈H₃₄O₆ 466.57 |
| 9 | 3'4'-OCH₃ | 3'4'-OCH₃ | 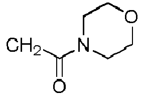 | H | 1 | C₂₉H₃₃NO₈ 523.57 |
| 10 | 3'4'-OCH₃ | 3'4'-OCH₃ | 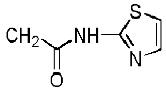 | H | 1 | C₂₈H₂₈N₂O₇S 536.60 |
| 11 | 3'4'-CH₃ | 3'4'-CH₃ | 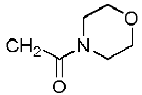 | CH₃ | 1 | C₃₀H₃₅NO₈ 537.60 |
| 12 | 3'4'-OCH₃ | 3'4'-OCH₃ | 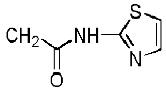 | CH₃ | 1 | C₂₉H₃₀N₂O₇S 550.62 |
| 13 | 3'4'-OCH₃ | 3'4'-OCH₃ | 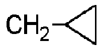 | F | 1 | C₂₇H₂₉FO₆ 468.51 |
| 14 | 3'4'-OOCH₃ | 3'4'-OCH₃ | 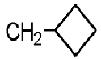 | F | 1 | C₂₈H₃₁FO₆ 482.54 |
| 15 | 3'4'-OCH₃ | 3'4'-OCH₃ | 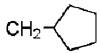 | F | 1 | C₂₉H₃₃FO₆ 496.57 |
| 16 | 3'4'-OCH₃ | 3'4'-OCH₃ | 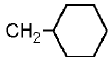 | F | 1 | C₃₀H₃₅FO₆ 510.59 |
| 17 | 3'4'-OCH₃ | 3'4'-OCH₃ | 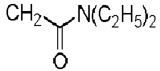 | F | 1 | C₂₉H₃₄FNO₇ 527.58 |
| 18 | 3'4'-OCH₃ | 3'4'-OCH₃ | 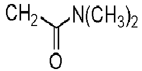 | F | 1 | C₂₇H₃₀FNO₇ 499.53 |
| 19 | 3'4'-OCH₃ | 3'4'-OCH₃ | (CH₂)₃CH₃ | F | 1 | C₂₇H₃₁FO₆ 470.53 |
| 20 | 3'4'-OCH₃ | 3'4'-OCH₃ | 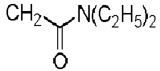 | Cl | 1 | C₂₉H₃₄ClNO₇ 544.04 |
| 21 | 3'4'-OCH₃ | 3'4'-OCH₃ | 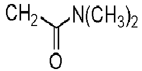 | Cl | 1 | C₂₇H₃₀ClNO₇ 515.98 |
| 22 | 3'4'-OCH₃ | 3'4'-OCH₃ | CH₂CH₂COOC₂H₅ | CH₃ | 1 | C₂₉H₃₄O₈ 510.58 |
| 23 | 3'4'-OCH₃ | 3'4'-OCH₃ | CH₂CH₂COOC₂H₅ | F | 1 | C₂₈H₃₁FO₈ 514.54 |
| 24 | 3'4'-OCH₃ | 3'4'-OCH₃ | CH₂CH₂COOH | CH₃ | 1 | C₂₇H₃₀O₈ 482.52 |
| 25 | 3'4'-OCH₃ | 3'4'-OCH₃ | CH₂CH₂COOH | F | 1 | C₂₆H₂₇FO₈ 486.49 |
| 26 | 3'OCH₃,4'OH | 3'OCH₃,4'OH | CH₂CH₂COOC₂H₅ | CH₃ | 1 | C₂₇H₃₀O₈ 482.52 |
| 27 | 3'OCH₃,4'OH | 3'OCH₃,4'OH | CH₂CH₂COOC₂H₅ | F | 1 | C₂₆H₂₇FO₈ 486.49 |
| 28 | 3'-OCH₃ | 3'-OCH₃ | 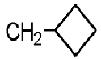 | CH₃ | | C₂₇H₃₀O₄ 418.52 |
| 29 | 3'-OH | 3'-OH | 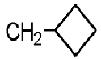 | CH₃ | 1 | C₂₅H₂₆O₄ 390.47 |
| 30 | 3'-OCH₃ | 3'-OSO₂C₂H₅ | 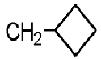 | CH₃ | 1 | C₂₈H₃₂O₆S 496.62 |
| 31 | 3'-OSO₂C₂H₅ | 3'-OSO₂C₂H₅ | 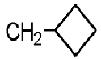 | CH₃ | 1 | C₂₉H₃₄O₈S₂ 574.71 |
| 32 | 3'4'-OCH₃ | 3'4'-OCH₃ | 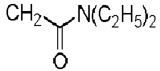 | CH₃ | 2 | C₃₄H₄₁NO₇ 575.69 |
| 33 | 3'4'-OCH₃ | 3'4'-OCH₃ | 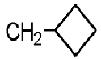 | F | 2 | C₃₂H₃₅FO₆ 534.62 |

In one more preferred embodiment of the invention, the compound is formula VIII, and R₁₈ is 3'4'-OCH₃; R₂₈ is 3'4'-OCH₃; R₃₈ is 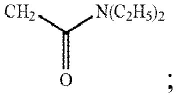 R₄₈ is F; and n₈ is 1, referred as JM17.

In one embodiment of the invention, the patient is suffering from an androgen receptor associated medical condition.

The term "androgen" as used herein refers to androgen hormones such as testosterone and dihydrotestosterone (DHT). DHT is the converted product of testosterone by the 5-alpha-reductase enzyme. Androgen stimulates or controls the development and maintenance of masculine characteristics and other physiological functions in vertebrates by binding to androgen receptors, which in turn bind to the androgen/AR-controlled genes (DNA) and activate or modulate the gene.

The term "androgen receptor" as used herein or "AR" refers to the intracellular receptor that specifically binds androgens, including testosterone and DHT. AR includes all mammalian isoforms, splice variants and polymorphisms of the androgen receptor.

In one embodiment of the invention, the androgen receptor associated medical condition is selected from inflammation, acne, alopecia, hirsutism, wound, spinal and bulbar muscular atrophy (SBMA, Kennedy's disease), unwanted immune response, immune disorder, or cancer.

Preferably, the cancer is selected from prostate cancer, liver cancer, bladder cancer, cervical cancer, lung cancer and breast cancer, skin cancer, small cell lung cancer, testicular cancer, lymphoma, leukemia, esophageal cancer, stomach cancer, colon cancer, endometrial cancer, ovarian cancer, central nervous system cancer, and the like.

According to the invention, in the selected patient who may respond to the compound, the compound may induce cytotoxicity against tumor cells or may inhibit tumor cell growth. In some embodiments, in the selected patient who may respond to the compound, activities of the compounds inhibit cancer cell growth or proliferation, optionally stimulated with a stimulator, such as DHT.

In other embodiments, in the selected patient who may respond to the compound, the compounds, their derivatives, pharmaceutical compositions and the like are used to prevent, treat or ameliorate symptoms from neurological and neuromuscular disorders such as Kennedy Disease. Spinal and bulbar muscular atrophy (SBMA), or Kennedy's disease, is a gender-specific motor neuron disease that affects 1 in every 40,000 males (reviewed by Katsuno et al., 2004). SBMA patients have a mutated androgen receptor containing an expanded polyglutamine tract. The expanded polyglutamine androgen receptor forms aggregates that interfere with cell functioning and is the factor that causes the proximal muscle atrophy associated with SBMA. In the selected patient who may respond to the compound, the compound may include relieving the stress caused by aggregate formation by reducing the amount of the mutant AR to a level that could be more easily policed by the cells' native housekeeping machinery. In the selected patient who may respond to the compound, the compound may including selectively degrading the androgen receptor and thus may be used as a therapy for SBMA. In the selected patient who may respond to the compound, the compounds that can enhance androgen receptor degradation may suppress the steady-state level of the receptor thus, attenuating the severity of aggregate formation in patients.

In the selected patient who may respond to the compound, the compound may prevent, treat or ameliorate symptoms from androgen related hair disorders. For example, androgenetic alopecia or "male pattern baldness" is hair loss caused by androgen activity on the androgen receptors in follicles and adjacent cells. As another example, hirsutism is excessive growth of thick, dark hair in locations where hair growth in women usually is minimal or absent. Such male-pattern growth of terminal body hair usually occurs in androgen-stimulated locations, such as the face, chest, and areolae.

In the selected patient who may respond to the compound, the compound may treat inflammation (e.g., rheumatoid arthritis), acne, alopecia, and may accelerate wound healing. Acne is caused by androgen-induced AR activation of sebaceous glands and may therefore be treated by administering a compound capable of preventing or decreasing AR activation. Compounds of the present invention are believed to induce degradation of the androgen receptor and thus would provide an effective treatment against such medical conditions. Androgenetic alopecia and other hair growth disorders are known to be caused by the activation of androgen receptors (AR) in hair follicles by endogenous androgen. Certain inflammation conditions and wound healing are also believed to be associated with the androgen receptor in response to androgen.

In the selected patient who may respond to the compound, the compound may be used in the treatment of endocrine disorders. Androgen excess is one of the most common endocrine disorders in women (reviewed by Bulun and Adashi, 2003). This pathophysiological status can be found in women with various endocrine disorders, including polycystic ovary syndrome (PCOS), pituitary adenoma-induced hyperprolactinemia, Cushing's syndrome, congenital adrenal hyperplasia, non-classical adrenal hyperplasia, ovary or adrenal tumor, and iatrogenic androgen excess. Among these disorders, PCOS, occurring in 5-10% of reproductive-age women, is the most frequently identified cause of hyperandrogenism. Recently, a relative increase in the ratio of circulating androgens to circulating estrogens (called androgenicity) has been observed in post-menopausal women (Lee et al., 2004). Androgenicity is the consequence of a greater decrease in estradiol and estrone synthesis than that of androgen synthesis after menopause, and its clinical implications are under active study. It has been shown that women exhibiting androgenicity are more frequently seen with central obesity (Peohlman et al., 1995). Fat deposit in the abdominal wall is metabolically active and is associated with insulin resistance in the peripheral tissues (Evans et al., 1983). Other than the above-mentioned endocrine disturbances, hyperandrogenic symptoms can also be detected in human immunodeficiency virus (HIV)-infected women showing lipodystrophy syndrome (Hadigan et al., 2000). It has been suggested that hyperandrogenism may be involved in lipid aberrations observed in the latter group of patients.

The method according to the invention comprises contacting the compound with a sample derived from the patient. Examples of the sample include but are not limited to biopsy, blood, tissues, urine, cells, excretions, or tissue fluid secretions. Preferably, the sample is derived from a biopsy of the cancer.

The method comprises contacting the compound with a sample derived from the patient and determining whether the compound decreases the amount of a protein selected from AR, c-MYC, Aurora-A, mutated p53, CDK4, or Her2 or whether the compound increases the amount of reactive oxygen species in the sample, wherein decrease of the protein or increase of the reactive oxygen species indicates that the patient may respond to the compound. Preferably, the method comprises determining whether the compound enhances the degradation of the protein.

The manner for determining the amount of the protein or the reactive oxygen species includes but is not limited to Western blotting, immunoprecipitation, or spectroscopic procedures. Preferably, the manner is based on antibody-antigen affinity.

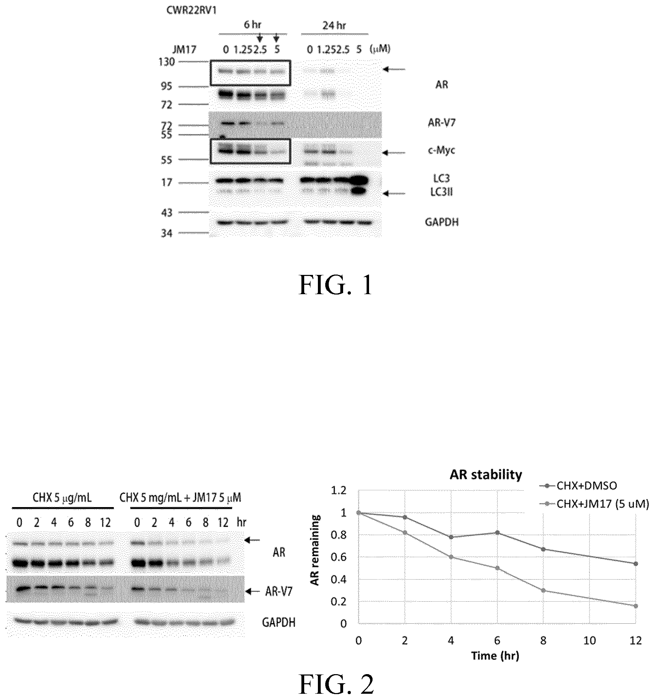

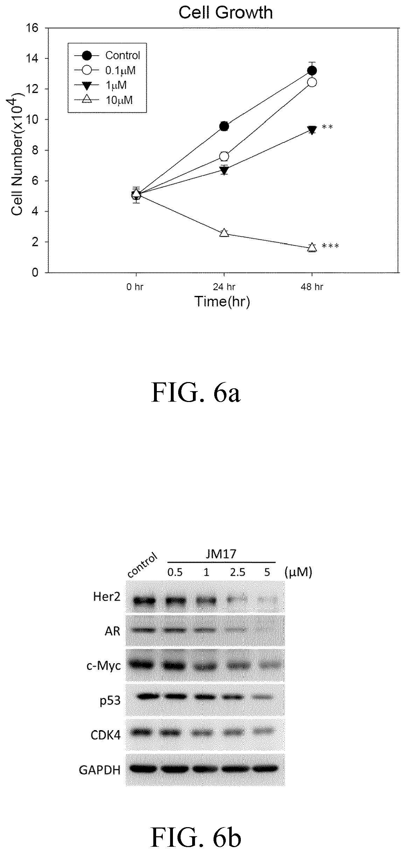

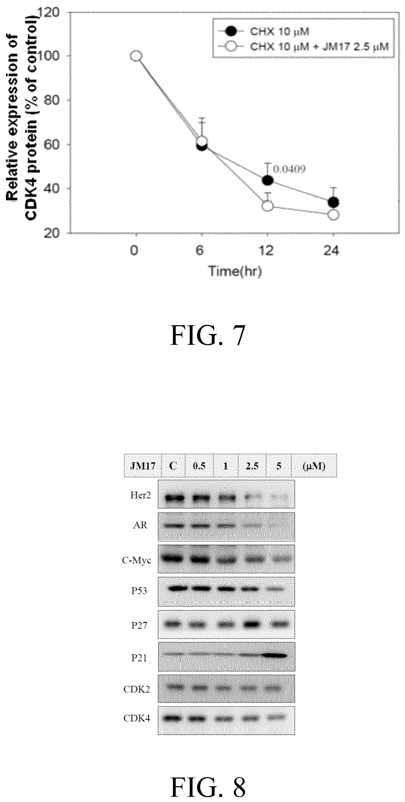

As shown in Examples, JM17 enhances AR, c-MYC and Aurora-A protein degradation in prostate cancer cells through ROS mediated pathway *in vitro* (FIGs. 1 to 5). More than AR, c-MYC and Aurora-A, CDK4, mutant p53 and Her2 were also degraded by JM17 treatment in BT-474 cell (a triple positive breast cancer cell line) *in vitro* (FIG. 6). To address whether JM17 declines half-life of CDK4 protein, cycloheximide was applied to block protein synthesis. The result shows JM17 significantly reduced the half-life of CDK4 protein in MDA-MB-231 cell (a triple negative breast cancer cell line) (FIG. 7).

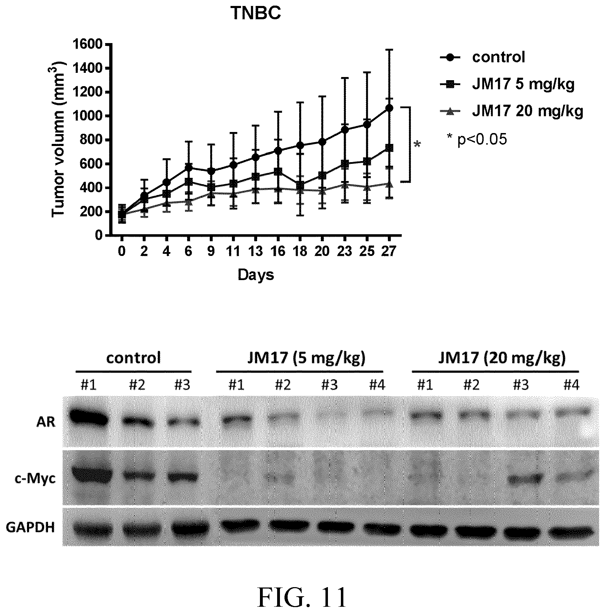

Per breast cancer xenograft model, JM17 decreases the tumor growth of MDA-MB-231 cells (a triple negative breast cancer cell line) along with reducing the protein expression of AR and c-MYC in xenograft (FIG. 11).

The invention also provides a kit comprising a compound according to formula I, IIa, IIb, IIc, III, IV, V, VI, VII or VIII and a detecting molecule for detecting the amount of a protein selected from AR, c-MYC, Aurora-A, mutated p53, CDK4, or Her2 or a detecting molecule for detecting the amount of reactive oxygen species.

Preferably, the detecting molecule is an antibody specific for AR, c-MYC, Aurora-A, mutated p53, CDK4, or Her2 or reactive oxygen species.

The invention also provides a method for identifying a candidate compound which may treat an androgen receptor associated medical condition, which comprises contacting the candidate compound with a sample derived from a patient suffering the androgen receptor associated medical condition and determining whether the candidate compound decreases the amount of a protein selected from AR, c-MYC, Aurora-A, mutated p53, CDK4, or Her2 or determining whether the compound increases the amount of reactive oxygen species in the sample, wherein decrease of the protein or increase of the reactive oxygen species indicates that the candidate compound may treat the androgen receptor associated medical condition such as cancer. Preferably, the androgen receptor is the protein indicated.

The invention also provides a kit comprising a sample derived from a patient suffering the androgen receptor associated medical condition and a detecting molecule for detecting the amount of a protein selected from AR, c-MYC, Aurora-A, mutated p53, CDK4, or Her2, or a detecting molecule for detecting the amount of reactive oxygen species.

The invention also provides a candidate compound identified by the method as mentioned above.

The following examples are provided to aid those skilled in the art in practicing the present invention.

### EXAMPLES

### JM17 enhances both AR and c-MYC protein degradation.

Per the NCI-60 cell lines screening, the IC50s were determined to be around ∼5µM. We have also studied the effect of JM17 on both AR and c-MYC protein levels in selected prostate cancer cell lines treated at 5 and 2.5 µM, respectively, for 6-hr (FIG. 1).

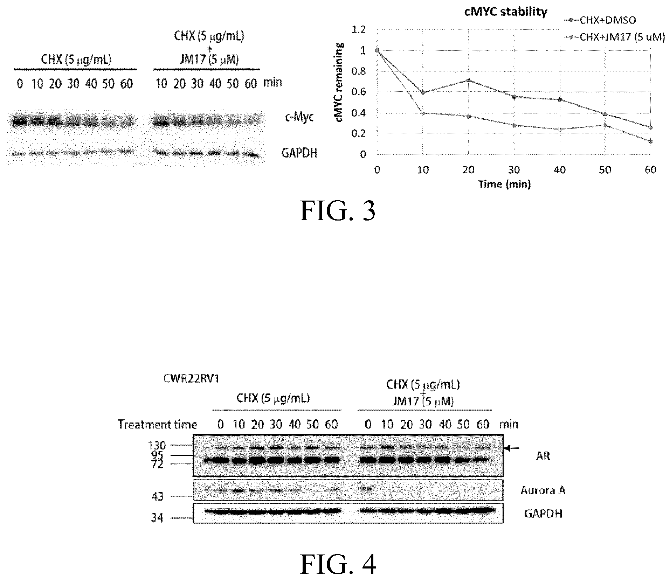

To address whether the declines of AR and c-MYC proteins after JM17 treatment were due to protein degradation, we performed "turnover" experiments in CWR22RV1 (prostate cancer cells) using cycloheximide, which blocked protein synthesis. Compared with the DMSO vehicle control, JM17 significantly reduced the half-life of AR protein from 12-hr to 6-hr (FIG. 2) and c-MYC protein from ∼45-min to ∼8-min (FIG. 3). In addition to AR and c-MYC, we also demonstrated JM17 enhanced Aurora A protein degradation (FIG.4).

### JM17 triggered the ROS mediated protein degradation in prostate cancer cells.

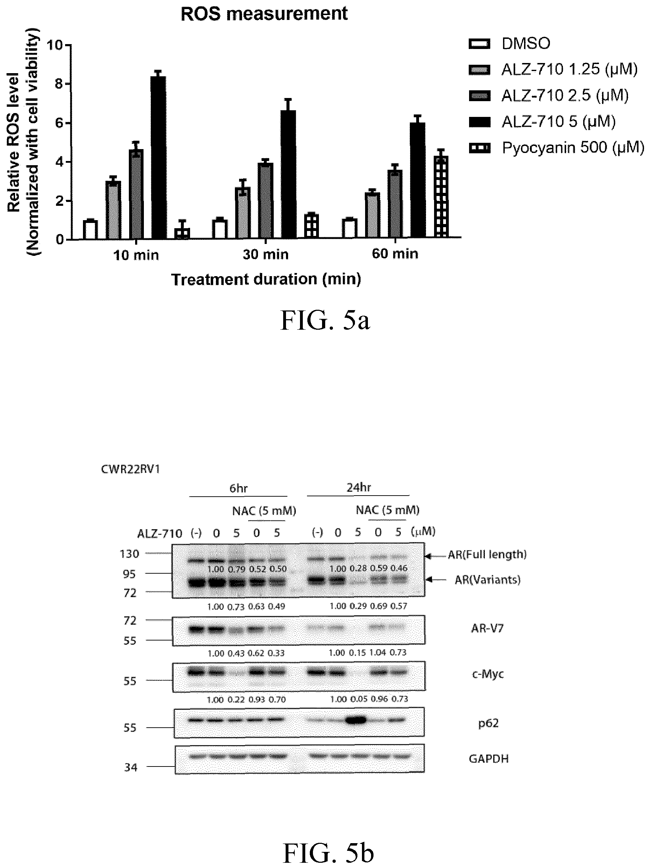

CWR22RV1 cells, a prostate cancer cell line, were treated with DMSO control or JM17 at the dose of 1.25, 2.5, and 5 µM. Then, the ROS level was detected at 10, 30, or 60 min after JM17 was added. The results are shown in FIG. 5. Compared with DMSO control, JM17 induced ROS within 60 min (FIG. 5a). After co-treatment with JM17 (5 µM) with or without NAC (a ROS scavenger, 5 mM), the expression level of AR, AR-V7, c-Myc and p62 was detected via western blot. The JM17 mediated protein degradation was blocked by NAC (FIG. 5b).

### JM17 suppressed BT-474 cell growth coupling with the decrease of protein expression on Her2, AR, c-MYC, mutant p53 and CDK4.

As shown in FIG. 6, the JM17 significantly reduced cell growth of BT-474, a triple positive breast cancer cell line, for 48 hr-treatment (FIG. 6a). The JM17 decreased the protein expression of Her2, AR, c-MYC, mutant p53 and CDK4 at the dose of 1 µM (FIG. 6b).

### JM17 enhanced CDK4 protein degradation.

MDA-MB-231 cells were treated with cycloheximide (CHX) with or without 2.5 µM JM17 and harvested at indicated time points for western blotting for CDK4 protein. The quantification of CDK4 band intensities, normalized to GAPDH, was calculated. As shown in FIG. 7, the JM17 significantly enhanced CDK4 protein degradation at 12-hr treatment.

### JM17 suppressed tumor growth in breast cancer xenograft model

To expand the application of JM17 to other cancer types, JM17 induced protein degradation was characterized in a breast cancer cell-line, BT-474, which is a triple-positive breast cancer cell-line with AR expression. In addition to AR and c-MYC, the immunoblotting result showed JM17 also reduced Her2, p53, and CDK4 protein expression (FIG. 8).

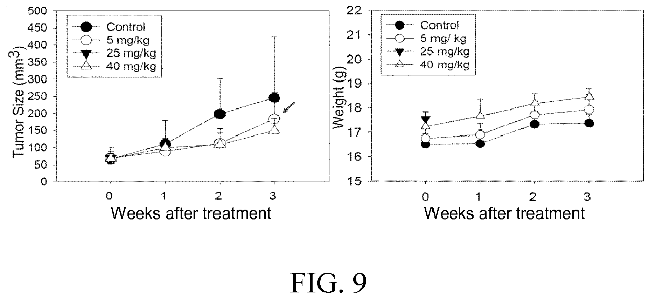

To further explore JM17 as an anti-cancer agent, we estimated anti-tumor efficacy of JM17 in subcutaneous breast cancer xenograft model. Compared with vehicle control of BT-474 tumor bearing mouse, both low (5 mg/kg) and high (40 mg/kg) doses of JM17 treatment groups decreased the tumor volume at week 3 (FIG. 9, left). Meanwhile, both JM17 groups showed an increase in body weight, meaning that neither 5 mg/kg nor 40 mg/kg of JM17 has any toxicity serious issue. (FIG. 9, right).

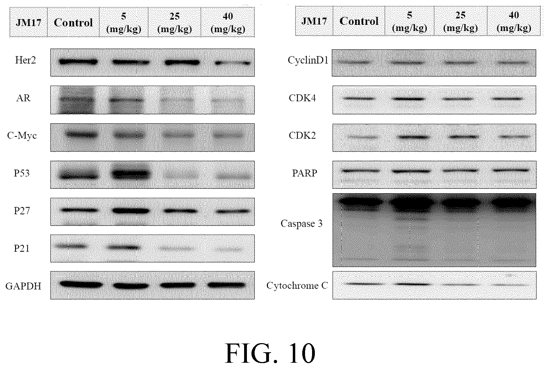

After mouse sacrifice, the protein expression of tumors was measured by immunoblotting with indicated antibodies (FIG. 10).

The effects of JM17 on the growth rate of MDA-MB-231 tumor in mice xenograft model are also shown in FIG. 11. The upper panel shows the average size of subcutaneous tumors after treatment with the vehicle (as the control), 5 mg/kg or 20 mg/kg of JM17, 3 times a week for 28 days. The JM17 significantly reduced the tumor size at the dose of 20 mg/kg. The tumors were isolated for protein extraction at 28 days after the vehicle, 5 mg/kg or 20 mg/kg of JM17 treatment (the lower panel). JM17 decreased the AR and c-MYC expression in tumor samples.

While the present invention has been described in conjunction with the specific embodiments set forth above, many alternatives thereto and modifications and variations thereof will be apparent to those of ordinary skill in the art. All such alternatives, modifications and variations are regarded as falling within the scope of the present invention.

## Claims

1. A method for selecting a patient who may respond to a compound according to formula I, IIa, IIb, IIc, III, IV, V, VI, VII or VIII, which comprises contacting the compound with a sample derived from the patient and determining whether the compound decreases the amount of a protein selected from AR, c-MYC, Aurora-A, mutated p53, CDK4, or Her2 or whether the compound increases the amount of reactive oxygen species in the sample, wherein decrease of the protein or increase of the reactive oxygen species indicates that the patient may respond to the compound; 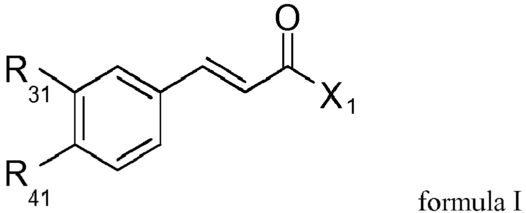 wherein in formula I:
R₃₁ and R₄₁ are each independently selected from the group consisting of alkoxy, hydroxy, and hydrogen; and
X₁ is selected from the group consisting of hydroxy, alkoxy, ethyl propionate, ethyl methyl carbonate, and carbonyl alkyl; 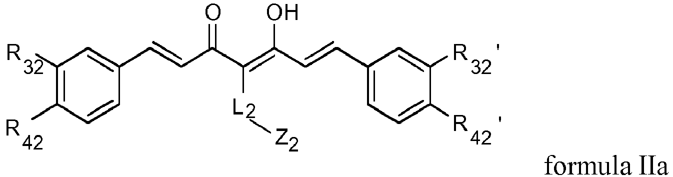 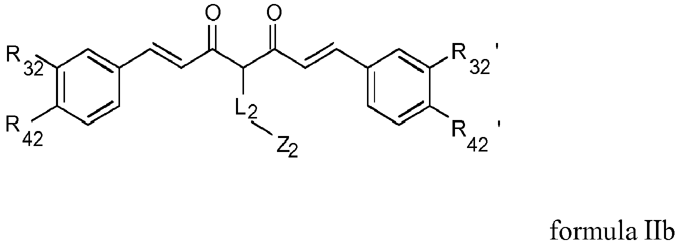
wherein in formula IIa and formula IIb:
R₃₂, R₄₂, R₃₂', and R₄₂' are independently selected from the group consisting of -H, -OH, and -OCH₃;
L₂ is a C0 - C8 alkylene; or L₂ is an unsaturated alkenylene or alkynl when Z₂ is nothing;
Z₂ is selected from the group consisting of -H, -OH, an aromatic ring, a cycloalkyl, -COR₁₂, -CO₂R₁₂, -CONR₁₂R₂₂, -NR₁₂R₂₂, -C(X₂)₃, wherein R₁₂ and R₂₂ are independently selected from the group consisting of -H, -CH₃, and -C₂H₅; and
X₂ is a halogen atom selected from the group consisting of -F, -Cl, and -Br; 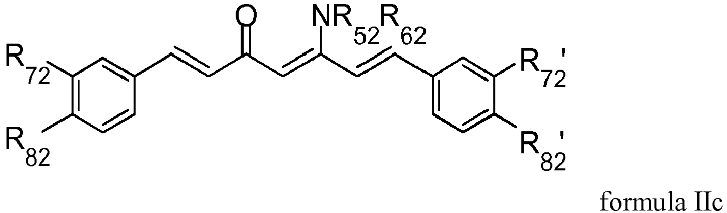
wherein in formula IIc:
R₇₂, R₈₂, R₇₂', and R₈₂' are independently selected from the group consisting of -H, -OH, and -OCH₃; and
R₅₂, and R₆₂ are independently selected from the group consisting of -H, -CH₃, -C₂H₅, a substituted aryl and a substituted benzyl group; 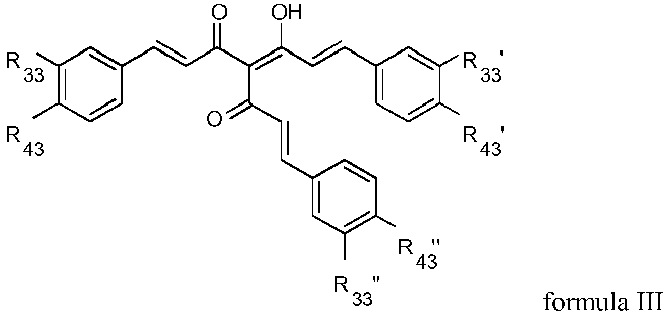
wherein in formula III:
R₃₃, R₄₃, R₃₃', R₄₃', R₃₃", and R₄₃" are each independently selected from the group consisting of alkoxy, hydroxy, and hydrogen; 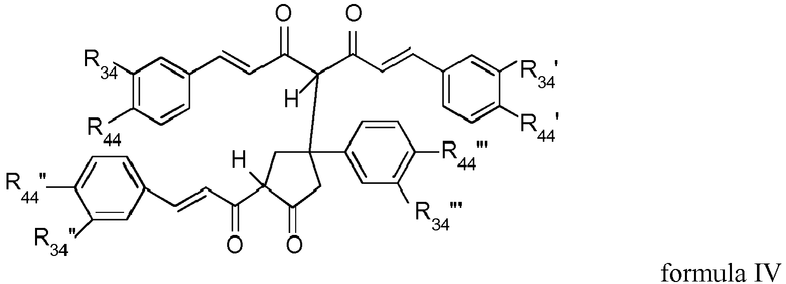 wherein in formula IV:
R₃₄, R₄₄, R₃₄', R₄₄', R₃₄", R₄₄", R₃₄'", and R₄₄'" are each independently selected from the group consisting of alkoxy, hydroxy, and hydrogen; 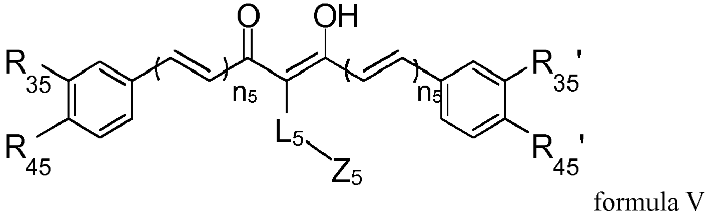 wherein in formula V:
each "ns" is independently 1, 2, or 3;
R₃₅, R₄₅, R₃₅', and R₄₅' are independently selected from the group consisting of -H, -OH, and -OCH₃;
L₅-Z₅ is absent; or
L₅-Z₅ exists, and L₅ is selected from the group consisting of a C0-C8 alkylene, an unsaturated alkenylene, and alkynl when Z₅ is absent;
Z₅ is selected from the group consisting of -H, -OH, an aromatic ring, a cycloalkyl, -COR₁₅, -CO₂R₁₅, -CONR₁₅R₂₅, -NR₁₅R₂₅, -C(X₅)₃;
R₁₅ and R₂₅ are independently selected from the group consisting of -H, -CH₃, and -C₂H₅; and
X₅ is a halogen atom selected from the group consisting of -F, -Cl, and -Br; 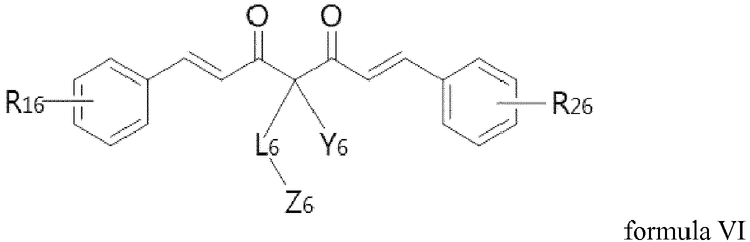 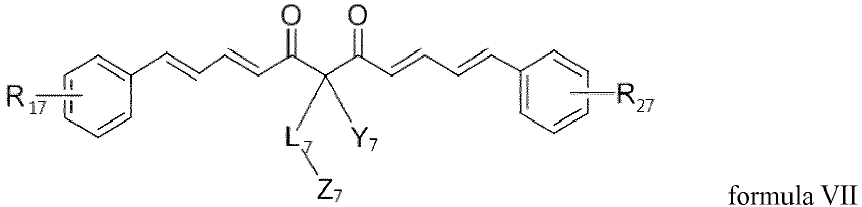
wherein in formula VI and formula VII:
R₁₆, R₁₇, R₂₆ and R₂₇ are mono- or di-substituted groups and independently selected from the group consisting of a methoxy group, a hydroxyl group, an alkyl sulfonyl group;
L₆ and L₇ are each selected from the group consisting of carbonyl, alkylene, alkenylene, and alkynl when Z₆ and Z₇ are absent;
Z₆ and Z₇ are each selected from the group consisting of -H, -OH, a substituted styrenyl, an aromatic ring, a cycloalkyl, -COR₃₆, -CONR₃₆R₄₆, and -C(X₆)₃, wherein R₃₆ and R₄₆ are each selected from the group consisting of -H, -CH₃, or -Cₙ₆H₂ₙ₆₊₁,, a heterocyclic, and a heteroaryl moiety; or R₃₆ and R₄₆ together form a heterocyclic ring; or
Z₆ and Z₇ are each COOR₆; wherein R₆ is selected from the group consisting of -H, -CH₃, and -Cₙ₇H₂ₙ₇₊₁; or
Z₆ and Z₇ are each a cycloalkyl when Y₇ is not H;
n₆ and n₇ are independently an integer from 2 to 4;
X₆ is selected from the group consisting of -F, -Cl, and -Br;
Y₆ and Y₇ are each H when L is an alkylene and Z₆ and Z₇ are -CONR₃₇R₄₇, R₃₇ and R₄₇ are each selected from the group consisting of -H, a hetercyclic, a heteroaryl,and a cycloalkyl; or
Y₆ and Y₇ are each selected from the group consisting of C1 - C3 alkyl, -F, Cl, and Br; and 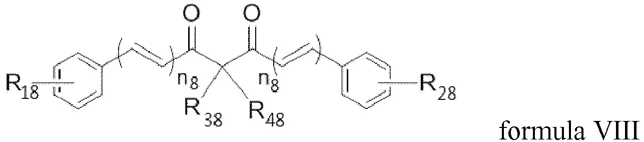
R₁₈ and R₂₈ are mono- or di-substituted groups and independently selected from the group consisting of a methoxy group, a hydroxyl group, and an alkyl sulfonyl group;
R₃₈ is selected from the group consisting of 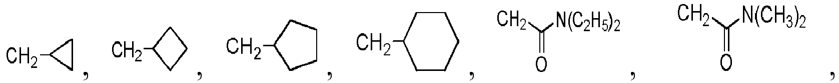 (CH₂)₃CH₃, 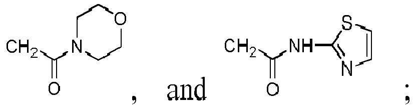 and
R₄₈ is selected from the group consisting of CH₃, H, F and Cl; and
n₈ is 1 or 2.

2. The method according to claim 1, wherein the patient is suffering from an androgen receptor associated medical condition.

3. The method according to claim 2, wherein the androgen receptor associated medical condition is selected from inflammation, acne, alopecia, hirsutism, wound, spinal and bulbar muscular atrophy (SBMA, Kennedy's disease), unwanted immune response, immune disorder, or cancer.

4. The method according to claim 3, wherein the cancer is selected from prostate cancer, liver cancer, bladder cancer, cervical cancer, lung cancer and breast cancer, skin cancer, small cell lung cancer, testicular cancer, lymphoma, leukemia, esophageal cancer, stomach cancer, colon cancer, endometrial cancer, ovarian cancer, central nervous system cancer, and the like.

5. The method according to at least one of claims 1 to 4, which comprises determining whether the compound enhances the degradation of the protein.

6. The method according to claim 3 or 4, wherein the sample is derived from a biopsy of the cancer.

7. The method according to at least one of claims 1 to 6, wherein the compound is formula VIII, and R₁₈ is 3'4'-OCH₃; R₂₈ is 3'4'-OCH₃; R₃₈ is 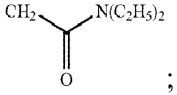 R₄₈ is F; and n₈ is 1.

8. A kit comprising a compound according to formula I, IIa, IIb, IIc, III, IV, V, VI, VII or VIII and a detecting molecule for detecting the amount of a protein selected from AR, c-MYC, Aurora-A, mutated p53, CDK4, or Her2, or a detecting molecule for detecting the amount of reactive oxygen species.

9. The kit according to claim 8, wherein the detecting molecule is an antibody specific for AR, c-MYC, Aurora-A, mutated p53, CDK4, or Her2 or reactive oxygen species.

10. The kit according to claim 8 or 9, wherein the compound is formula VIII, and R₁₈ is 3'4'-OCH₃; R₂₈ is 3'4'-OCH₃; R₃₈ is 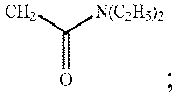 R₄₈ is F; and n₈ is 1.

11. A method for identifying a candidate compound which may treat an androgen receptor associated medical condition, which comprises contacting the candidate compound with a sample derived from a patient suffering the androgen receptor associated medical condition and determining whether the candidate compound decreases the amount of a protein selected from AR, c-MYC, Aurora-A, mutated p53, CDK4, or Her2 or determining whether the compound increases the amount of reactive oxygen species in the sample, wherein decrease of the protein or increase of the reactive oxygen species indicates that the candidate compound may treat the androgen receptor associated medical condition.

12. The method according to claim 11, wherein the androgen receptor associated medical condition is selected from inflammation, acne, alopecia, hirsutism, wound, spinal and bulbar muscular atrophy (SBMA, Kennedy's disease), unwanted immune response, immune disorder, or cancer.

13. The method according to claim 12, wherein the cancer is selected from prostate cancer, liver cancer, bladder cancer, cervical cancer, lung cancer and breast cancer, skin cancer, small cell lung cancer, testicular cancer, lymphoma, leukemia, esophageal cancer, stomach cancer, colon cancer, endometrial cancer, ovarian cancer, central nervous system cancer, and the like.

14. The method according to at least one of claims 11 to 13, which comprises determining whether the compound enhances the degradation of the protein.

15. The method according to at least one of claims 11 to 14, wherein the sample is derived from a biopsy of the cancer.

16. A kit comprising a sample derived from a patient suffering the androgen receptor associated medical condition and a detecting molecule for detecting the amount of a protein selected from AR, c-MYC, Aurora-A, mutated p53, CDK4, or Her2, or a detecting molecule for detecting the amount of reactive oxygen species.

17. The kit according to claim 16, wherein the androgen receptor associated medical condition is selected from inflammation, acne, alopecia, hirsutism, wound, spinal and bulbar muscular atrophy (SBMA, Kennedy's disease), unwanted immune response, immune disorder, or cancer.

18. The kit according to claim 17, wherein the cancer is selected from prostate cancer, liver cancer, bladder cancer, cervical cancer, lung cancer and breast cancer, skin cancer, small cell lung cancer, testicular cancer, lymphoma, leukemia, esophageal cancer, stomach cancer, colon cancer, endometrial cancer, ovarian cancer, central nervous system cancer, and the like.

19. The kit according to at least one of claims 16 to 18, wherein the sample is derived from a biopsy of the cancer.

20. The kit according to at least one of claims 16 to 19, wherein the detecting molecule is an antibody specific for AR, c-MYC, Aurora-A, mutated p53, CDK4, or Her2 or reactive oxygen species.
